(19) Europäisches Patentamt
European Patent Office
Office européen des brevets

(11) Veröffentlichungsnummer: **0 126 300 B1**

(12) # EUROPÄISCHE PATENTSCHRIFT

(45) Veröffentlichungstag der Patentschrift: **01.04.87**

(51) Int. Cl.⁴: **C 07 C 118/00**, C 07 C 119/045 // C07C125/073

(21) Anmeldenummer: **84104353.2**

(22) Anmeldetag: **17.04.84**

(54) **Mehrstufenverfahren zur Herstellung von 3-Isocyanatomethyl-3,5,5-trimethyl-cyclohexylisocyanat.**

(30) Priorität: **23.04.83 DE 3314790**

(43) Veröffentlichungstag der Anmeldung: **28.11.84 Patentblatt 84/48**

(45) Bekanntmachung des Hinweises auf die Patenterteilung: **01.04.87 Patentblatt 87/14**

(84) Benannte Vertragsstaaten: **BE DE FR GB IT NL**

(56) Entgegenhaltungen:
**EP - A - 0 018 588**
**EP - A - 0 061 013**

(73) Patentinhaber: **BASF Aktiengesellschaft, Carl-Bosch-Strasse 38, D-6700 Ludwigshafen (DE)**

(72) Erfinder: **Hellbach, Hans, Stettiner Strasse 14, D-6840 Lampertheim (DE)**
Erfinder: **Merger, Franz, Dr., Max-Slevogt-Strasse 25, D-6710 Frankenthal (DE)**
Erfinder: **Towae, Friedrich, Dr., Schwedlerstrasse 118, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 126 300 B1

**Beschreibung**

3-Isocyanatomethyl-3,5,5-trimethyl-cyclohexylisocyanat, im folgenden auch Isophoron-diisocyanat oder abgekürzt IPDI genannt, wird zur Zeit ausschliesslich hergestellt durch Phosgenierung von 3-Aminomethyl-3,5,5-trimethyl-cyclohexalamin, im folgenden auch als Isophoron-diamin oder abgekürzt IPDA bezeichnet, und anschliessende thermische Spaltung des intermediär gebildeten Isophoron-dicarbamidsäurechlorids in IPDI und Chlorwasserstoff.

Problematisch bei dieser Verfahrensweise sind der hohe Umsatz von Chlor über Phosgen und Carbamidsäurechorid in Chlorwasserstoff, die Toxizität des Phosgens sowie die damit verbundenen kostspieligen Sicherheitsprobleme, die Korrosivität des Reaktionsgemisches und die Labilität der in der Regel eingesetzten Lösungsmittel.

Es hat daher nicht an Versuchen gefehlt, Isocyanate, vorzugsweise aromatische Di- und/oder Polyisocyanate, phosgenfrei herzustellen.

Zur Herstellung von aliphatischen und/oder cycloaliphatischen Di- und/oder Polyurethanen werden gemäss EP-A-0018588 primäre aliphatische und/oder cycloaliphatische Di- und/oder Polyamine mit O-Alkylcarbamidsäureestern in Gegenwart von Alkoholen im Verhältnis von $NH_2$-Gruppen der Amine zu Carbamidsäureestern zu Alkohol von 1:0,8 bis 10:0,25 bis 50 bei Temperaturen von 160 bis 300°C in Gegenwart oder Abwesenheit von Katalysatoren umgesetzt und der entstehende Ammoniak gegebenenfalls abgetrennt. Die erhaltenen Di- und/oder Polyurethane können gegebenenfalls in die entsprechenden Di- und/oder Polyisocyanate übergeführt werden. Detaillierte Reaktionsbedingungen zur Thermolyse werden in der Publikation nicht offenbart.

Nach Angaben der EP-A 28338 werden aromatische Di- und/oder Polyisocyanate hergestellt nach einem Zweistufenverfahren, wobei in der ersten Reaktionsstufe primäre aromatische Di- und/oder Polyamine mit O-Alkyl-carbamidsäureestern in Abwesenheit oder Gegenwart von Katalysatoren und gegebenenfalls Harnstoff und Alkohol zu Aryl-di- und/oder -plyurethanen umgesetzt werden und das dabei gebildete Ammoniak gegebenenfalls abgetrennt wird und die erhaltenen Aryl-di- und/oder -polyurethane durch thermische Spaltung in der zweiten Reaktionsstufe in aromatischer Di- und/oder Polyisocyanate übergeführt werden.

Auf diese Weise können aromtische Di- und/oder Polyisocyanate ohne Mitverwendung von Phosgen in hohen Ausbeuten hergestellt werden.

Die DE-OS 3108990 beschreibt die Herstellung von IPDI durch thermische Spaltung unter Druck von 3-Ethoxicarbonylaminomethyl-3,5,5-trimethyl-1-ethoxicarbonylamino-cyclohexan in Gegenwart von Dibenzyltoluol als Lösungsmittel und eines Katalysatorgemisches aus Toluolsulfonsäuremethylester und Diphenylzinndichlorid. Angaben über die Gewinnung der Ausgangskomponente, deren Isolierung sowie die Reinigung und gegebenenfalls Rückgewinnung des Lösungsmittels und der Katalysatormischung werden nicht gemacht. Berechnungen über die Wirtschaftlichkeit des Verfahrens sind daher nicht möglich.

Aufgabe der vorliegenden Erfindung war es, IPDI mit hoher Selektivität in grossen Raum-Zeit-Ausbeuten kostengünstig auf einfache Weise ohne Verwendung von kostspieligen oder sicherheitsgefährdenden Ausgangs- oder Hilfsstoffen herzustellen.

Diese Aufgabe wurde gelöst durch ein mehrstufiges Verfahren zur Herstellung von IPDI, das dadurch gekennzeichnet ist, dass man

a) IPDA mit Harnstoff und Alkohol in Gegenwart von Dialkylcarbonaten und/oder Carbamidsäurealkylestern sowie gegebenenfalls Katalysatoren zu 3-Alkoxicarbonylaminomethyl-3,5,5-trimethyl-1-alkoxicarbonylamino-cyclohexan umsetzt und das entstehende Ammoniak gleichzeitig abtrennt,

b) aus der erhaltenen Reaktionsmischung den Alkohol, die Dialkylcarbonate und/oder Carbamidsäurealkylester abtrennt und vorzugsweise in die Reaktionsstufe (a) zurückführt,

c) das 3-Alkoxicarbonylaminomethyl-3,5,5-trimethyl-1-alkoxicarbonylamino-cyclohexan in einem Verdampfer bei Temperaturen von 200 bis 300°C und einem Druck von 0,1 bis 200 mbar verdampft,

d) die Dämpfe bei Temperaturen von über 300°C und einem Druck von 0,1 bis 200 mbar in einem Spaltreaktor in IPDI und Alkohol thermisch spaltet und

e) die Spaltprodukte fraktioniert kondensiert.

Nach einer bevorzugten Ausführungsform des Verfahrens wird die erhaltene Reaktionsmischung (b) aus 3-Alkoxicarbonylaminomethyl-3,5,5-trimethyl-1-alkoxicarbonylamino-cyclohexan, Dialkylcarbonat und/oder Carbamidsäurealkylester, Alkohol und gegebenenfalls Isophoron-oligoharnstoff-polyurethanen zweistufig getrennt, wobei

i) in der ersten Stufe der Alkohol bis auf einen Restalkoholgehalt von 1 bis 30 Gew.%, bezogen auf das Gesamtgewicht der Reaktionsmischung (b) abdestilliert und in die Reaktionsstufe (a) zurückgeführt wird und

ii) in der zweiten Stufe der restliche Alkohol, das Dialkylcarbonat und/oder der Carbamidsäurealkylester durch Strippen mit Inertgas vom 3-Alkoxicarbonylaminomethyl-3,5,5-trimethyl-1-alkoxicarbonylamino-cyclohexan und gegebenenfalls Isophoron-oligoharnstoff-polyurethan abgetrennt und in die Reaktionsstufe (a) zurückgeführt werden.

Nach dem erfindungsgemässen Verfahren kann IPDI technisch problemlos mit sehr guten Ausbeuten hergestellt werden. Vorteilhaft bei dem Mehrstufenverfahren ist insbesondere, dass die eingesetzten und intermediär gebildeten Dialkylcarbonate und/oder Carbamidsäurealkylester und der Alkohol ohne zusätzliche kostspielige Reinigungs- und Rückgewinnungsprozesse in die Reaktionsstufe (a) zurückgeführt und wiederverwendet werden können.

Rein formal betrachtet, kann somit das erfindungsgemässe Verfahren schematisch durch folgende Gleichung bilanziert werden:

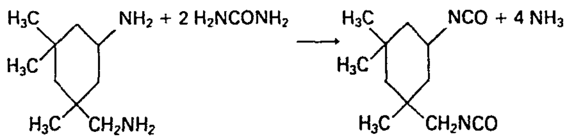

Zur Herstellung des 3-Alkoxicarbonylaminomethyl-3,5,5-trimethyl-1-alkoxicarbonylamino-cyclohexans in der Reaktionsstufe (a) wird IPDA mit Harnstoff und Alkohol im Molverhältnis 1:1,8 bis 2,5:2 bis 10, vorzugsweise 1:2,0 bis 2,3:3 bis 6, in Abwesenheit oder in Gegenwart von Katalysatoren bei Reaktionstemperaturen von 160 bis 300°C, vorzugsweise 180 bis 250°C und insbesondere 185 bis 240°C und unter einem Druck, der in Abhängigkeit von dem verwendeten Alkohol zwischen 0,1 bis 60 bar, vorzugsweise 1 bis 40 bar liegt, zur Reaktion gebracht. Für diese Reaktionsbedingungen ergeben sich Reaktionszeiten von 0,5 bis 50, vorzugsweise 3 bis 15 Stunden.

Als Alkohole eignen sich prinzipiell alle aliphatischen Alkohole. Vorzugsweise wird man jedoch solche auswählen, deren Siedepunkte genügend weit vom Siedepunkt des durch die thermische Spaltung erhaltenen IPDI entfernt liegen, so dass einerseits eine möglichst quantitative Trennung der Spaltprodukte IPDI und Alkohol möglich ist und andererseits die erhaltenen 3-Alkoxicarbonylaminomethyl-3,5,5-trimethyl-1-alkoxicarbonyl-cyclohexane, gegebenenfalls neben Isophoron-oligoharnstoff-polyurethanen, möglichst unzersetzt verdampft werden können.

Aus diesen Gründen verwendet werden daher vorzugsweise Alkohole, wie z.B. Methanol, Ethanol, n-Propanol, n-Butanol, iso-Butanol, n-Pentanol, iso-Pentaol, n-Hexanol, oder Gemische der genannten Alkohole und insbesondere n-Propanol, n- und/oder iso-Butanol.

Wie bereits dargelegt wurde, wird die Umsetzung in der Reaktionsstufe (a) in Gegenwart von Dialkylcarbonaten in einer Menge von 1 bis 30 Mol.%, vorzugsweise 5 bis 25 Mol.% oder Carbamidsäurealkylestern in einer Menge von 1 bis 20 Mol.%, vorzugsweise von 5 bis 18 Mol.%, bezogen und IPDA durchgeführt. Vorzugsweise verwendet werden jedoch Mischungen aus Dialkylcarbonaten und Carbamidsäurealkyestern in den genannten Mengenverhältnissen. Als Dialkylcarbonate und/oder Carbamidsäureester setzt man bevorzugt solche ein, deren Alkylreste dem Alkylrest des verwendeten Alkohols entsprechen.

Zur Erhöhung der Reaktionsgeschwindigkeit können die 3-Alkoxicarbonylaminomethyl-3,5,5-trimethyl-1-alkoxicarbonylamino-cyclohexane in Gegenwart von Katalysatoren hergestellt werden. Diese werden zweckmässigerweise in Mengen von 0,1 bis 20 Gew.%, vorzugweise 0,5 bis 10 Gew.%, und insbesondere 1 bis 5 Gew.%, bezogen auf das Gewicht des IPDA, verwendet. Als Katalysatoren eignen sich anorganische oder organische Verbindungen, die ein oder mehrere Kationen, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäss Handbook of Chemistry and Physics 14th. Edition, publiziert von Chemical Rubber Publishing Co., 23 Superior Ave. N.E., Cleveland, Ohio, enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxilate, Chelate, Carbonate und Thio- oder Dithiocarbamate. Beispielhaft genannt seien die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen, Cobalt und Nickel. Vorzugsweise Verwendung finden die Kationen von Lithium, Calcium, Aluminium, Zinn, Bismut, Antimon, Kupfer, Zink, Titan, Vanadium, Chrom, Molybdän, Mangan, Eisen und Cobalt. Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumethanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminium-isobutylat, Aluminiumtrichlorid, Bismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexalat, Zinkbenzoat, Zinkundecylenat, Cer-(IV)-oxid, Uranylacetat, Titantetrabutanolat, Titantetrachlorid, Titantetraphenolat, Titannaphthenat, Vanadium-(III)-chlorid, Vanadiumacetonylacetat, Chrom-(III)-chlorid, Molybdän-(VI)-oxid, Molybdänacetylacetonat, Wolfram-(VI)-oxid, Mangan-(II)-chlorid, Mangan-(II)-acetat, Mangan-(III)-acetat, Eisen-(II)-acetat, Eisen-(III)-acetat, Eisenphosphat, Eisenoxalat, Eisen-(III)-chlorid, Eisen-(III)-bromid, Cobaltacetat, Cobaltchlorid, Cobaltsulfat, Cobaltnaphthenat, Nickelchlorid, Nickelacetat und Nickelnaphthenat sowie deren Gemische.

Es hat sich als vorteilhaft erwiesen, das entstehende Ammoniak sofort aus der Reaktionsmischung, beispielsweise durch Destillation, abzutrennen. Die hierfür verwendete Vorrichtung, beispielsweise eine Destillatonskolonne, wird bei Temperaturen von 60 bis 150°C, vorzugsweise 65 bis 120°C betrieben, so dass eine Belegung durch Ammoniumcarbaminat, welches in minimalen Mengen aus Ammoniak und Kohlendioxid durch Zersetzung von Harnstoff gebildet wird, vermieden werden kann.

Nach beendeter Reaktion werden aus der erhaltenen Reaktionsmischung (b) der Alkohol, die Dialkylcarbonate und/oder Carbamidsäurealkylester abgetrennt und zur Wiederverwendung bei nachfolgenden Ansätzen bereitgehalten; bei kontinuierlicher Fahrweise werden sie jedoch vorzugsweise direkt in die Reaktionsstufe (a) zurückgeführt.

Wie bereits oben dargelegt wurde, wird die Abtrennung der genannten Verbindungen vorzugsweise zweistufig durchgeführt. In der ersten Stufe wird der Alkohol bis auf einen Restalkoholgehalt von 1 bis 30 Gew.%, vorzugweise 2 bis 15 Gew.%, bezogen auf das Gewicht der Reaktionsmischung (b) abdestilliert und in die Reaktionsstufe (a) zurückgeführt.

Die aufkonzentrierte Reaktionsmischung (b), die zum grössten Teil aus 3-Alkoxicarbonylaminomethyl-3,5,5-trimethyl-1-alkoxicarbonylamino-cyclohexan und gegebenenfalls Isophoron-oligoharnstoff-polyurethanen besteht und noch den restlichen Alkohol, Dialkylcarbonat und/oder Carbamidsäurealkylester enthält, wird in der zweiten Stufe in einer Strippkolonne mit 50 bis 5000 Liter, vorzugsweise 100 bis 1000 Liter Inertgas pro Liter aufkonzentrierter Reaktionsmischung (b) und Stunde bei Stripptemperaturen von 50 bis 200°C, vorzugsweise 120 bis 180°C behandelt, um den restlichen Alkohol, die Dialkylcarbonate und/oder Carbamidsäurealkylester praktisch vollständig abzutrennen. Geeignete Inertgase hierfür sind beispielsweise Stickstoff, Kohlenmonoxid, Edelgase und Erdgas. Die abgestrippten leichtersiedenden Verbindungen werden kondensiert, gegebenenfalls zwischengelagert und zur Wiederverwendung bei weiteren Ansätzen bereitgehalten. Bei kontinuierlicher Fahrweise werden sie vorzugsweise direkt in die Reaktionsstufe (a) zurückgeführt.

Der nach der Strippung erhaltene Rückstand (c), der im wesentlichen aus 3-Alkoxicarbonylaminomethyl-3,5,5-trimethyl-1-alkoxicarbonylamino-cyclohexan und gegebenenfalls Isophoron-oligoharnstoff-polyurethanen besteht, kann in flüssiger oder fester Form oder auch als Suspension oder Lösung in einem unter den Reaktionsbedingungen inerten Lösungsmittel in einem Verdampfer und einem nachfolgenden Spaltreaktor thermisch gespalten werden.

Nach der bevorzugten Ausführungsform des erfindungsgemässen Verfahrens wird der Rückstand (c) lösungsmittelfrei, in Form einer auf 80 bis 180°C, vorzugsweise 100 bis 150°C erhitzten Schmelze mit einer Dosierpumpe in den Verdampfer eingebracht.

Als Verdampfer, die bei Temperaturen von 200 bis 300°C, vorzugsweise von 220 bis 300°C und insbesondere von 240 bis 280°C und unter einem Druck von 0,1 bis 200 mbar, vorzugsweise von 5 bis 100 mbar betrieben werden, haben sich insbesondere Dünnschichtverdampfer oder Wirbelschichtverdampfer bewährt. Es können jedoch auch beliebige andere Verdampfer verwendet werden, z.B. Schneckenverdampfer, A.P.-Reaktoren (Hersteller: Krauss-Maffei), Metallschlangen- und Rührbett-Verdampfer.

Bei Verwendung von Dünnschichtverdampfern ist es zwar durch eine ausreichende Wärmezufuhr möglich, das gesamte zugeführte 3-Alkoxicarbonylaminomethyl-3,5,5-trimethyl-1-alkoxicarbonylamino-cyclohexan zu verdampfen. Vorteilhafterweise wird jedoch ein Teil des zugeführten 3-Alkoxicarbonylaminomethyl-3,5,5-trimethyl-1-alkoxicarbonylamino-cyclohexan gemeinsam mit gegebenenfalls vorhandenem Isophoron-oligoharnstoff-polyurethan unverdampft als Schmelze aus dem Verdampfer ausgeschleust, da man hierdurch einen bedeutenden Reinigungseffekt an der Verdampferwand erzielt. Das Gewichtsverhältnis von verdampften zu unverdampften 3-Alkoxicarbonylaminomethyl-3,5,5-trimethyl-1-alkoxicarbonylamino-cyclohexan kann in breiten Grenzen, beispielsweise von 20:80 bis 90:10 variiert werden. Die aus dem Verdampfer ausgeschleuste Schmelze wird vorzugsweise direkt in die Reaktionsstufe (a), die Diurethanisierungsstufe, zurückgeführt.

Die 3-Alkoxicarbonylaminomethyl-3,5,5-trimethyl-1-alkoxicarbonylamino-cyclohexan-Dämpfe (d) werden in den Spaltreaktor eingebracht und bei einer Temperatur von über 300°C, vorzugsweise 310 bis 480°C und insbesondere 360 bis 440°C und unter vermindertem Druck, beispielsweise von 0,1 bis 200 mbar, vorzugsweise 0,1 bis 100 mbar und insbesondere 1 bis 50 mbar, diskontinuierlich oder vorzugsweise kontinuierlich in IPDI und Alkohol thermisch gespalten.

Der Spaltreaktor, der im allgemeinen säulenförmig ist, kann einen Querschnitt in beliebiger Form aufweisen. Vorzugsweise verwendet man langgestreckte, zylinderförmige Spaltreaktoren. Das Verhältnis von Innendurchmesser zu Länge des Spaltreaktors beträgt im allgemeinen 1:2 bis 1:1000, vorzugsweise 1:10 bis 1:500. Die Spaltreaktoren können senkrecht oder waagrecht ausgerichtet sein und auch Zwischenlagen einnehmen. Vorzugsweise verwendet werden Röhrenöfen als Spaltreaktoren, bei denen der Rohrinnendurchmesser etwa 10 bis 100 mm und die Rohrlänge ungefähr 0,5 bis 5 m beträgt.

Zweckmässigerweise führt man die Spaltung in Gegenwart von thermisch stabilen Reaktorfüllkörpern durch. Als Füllkörper geeignet sind alle temperaturbeständigen und gasdurchlässigen Materialien, wie z.B. Perlen, Wolle, Ringe und/oder Späne aus Kohle, Stahl, Messing, Kupfer, Zink, Aluminium, Titan, Chrom, Cobalt, Nickel und/oder Quarz. Einige dieser Materialien, wie Stahl, Messing, Aluminium und Zink, haben sich besonders bewährt und werden daher bevorzugt verwendet, da sie zu besseren Spaltergebnissen führen. Hierbei ist noch ungeklärt, ob es sich um katalytische oder physikalische Effekte, wie beispielsweis eine bessere Wärmeübertragung, handelt oder ob eine synergistische Kombination beider Effekte vorliegt.

Aus dem Spaltreaktor führt man die in der Dampfphase befindlichen Dissoziationsprodukte, die nahezu ausschliesslich aus IPDI und Alkohol bestehen, in eine Zweistufen-Dampfkondensationsvorrichtung (e). In der ersten Kondensationsstufe, die abhängig vom Systemdruck von 0,1 bis 100 mbar bei Temperaturen von 60 bis 120°C betrieben wird, kondensiert das IPDI nahezu vollständig aus.

Bei Verwendung des bevorzugt eingesetzten 3-Butoxicarbonylaminomethyl-3,5,5-trimethyl-1-butoxicarbonylamino-cyclohexan wird bei einem Systemdruck von 20 bis 40 mbar zweckmässigerweise eine Kondensationstemperatur von 70 bis

100°C eingehalten. In der zweiten Kondensationsstufe wird im wesentlichen Alkohol kondensiert, der in die Reaktionsstufe (a) zurückgeführt wird. Die Temperatur der zweiten Kondensationsstufe richtet sich nach dem Siedepunkt des zu kondensierenden Alkohols. Bei der Spaltung von 3-Butoxicarbonylaminomethyl-3,5,5-trimethyl-1-butoxicarbonylamino-cyclohexan wird zweckmässigerweise beim obengenannten Systemdruck eine Kondensationstemperatur von 5 bis 30°C eingehalten. Das in der ersten Kondensationsstufe erhaltene IPDI wird üblicherweise einer Reindestillation unterworfen und besitzt danach eine Reinheit von über 99,5 Gew.%. Das hierbei anfallende Sumpfprodukt wird ebenfalls in die Reaktionsstufe (a) zurückgeführt.

Je nach Wahl der Kondensationstemperaturen und in Abhängigkeit von Systemdruck, können in der ersten Kondensationsstufe Alkohol und in der zweiten Kondensationsstufe IPDI in unterschiedlichen Mengen mitkondensiert werden. Nach einer bevorzugten Ausführungsform lässt man in der zweiten Kondensationsstufe mitkondensiertes IPDI mit überschüssigem Alkohol zu 3-Alkoxicarbonylaminomethyl-3,5,5-trimethyl-1-alkoxicarbonylamino-cyclohexan abreagieren und führt es nach dem Abtrennen von Alkohol erneut der Verdampfung und Spaltung zu. Es ist jedoch auch möglich nach einer ebenfalls bevorzugten Ausführungsform, das 3-Alkoxicarbonylaminomethyl-3,5,5-trimethyl-1-alkoxicarbonylamino-cyclohexan zusammen mit dem Dialkylcarbonat und/oder Carbamidsäurealkylester in die Reaktionsstufe (a) zurückzuführen.

Auf analoge Weise lässt man den in der ersten Kondensationsstufe mitkondensierten Alkohol mit überschüssigem IPDI abreagieren und führt die Reaktionsprodukte nach der destillativen Abtrennung von IPDI der Verdampfung und Spaltung zu oder in der bevorzugten Ausführungsform unter Vermischung mit dem in der zweiten Kondensationsstufe erhaltenen Alkohol in die Reaktionsstufe (a) zurück.

Das nach dem erfindungsgemässen Verfahren hergestellte IPDI eignet sich vorzüglich zur Herstellung von Polyurethan- oder Polyurethan-Polyharnstoff-Kunststoffen und insbesondere für lichtbeständige Polyurethanlacke und -überzüge.

Beispiel

In einen Rührkessel mit aufgesetzter beheizter Kolonne und Druckhalteventil wurden 1700 g Isophorondiamin, 1200 g Harnstoff und 370 g n-Butanol eingefüllt. Zusätzlich gab man zu dieser Mischung 105 g Dibutylcarbonat, 117 g Carbamidsäurebutylester, 956 g 3-Butoxicarbonylaminomethyl-3,5,5-trimethyl-1-butoxicarbonylamino-cyclohexan und 3288 g n-Butanol, die man aus einem in gleicher Weise zuvor ausgeführten Versuch gewonnen hatte. Diese Reaktionsmischung wurde 10 Stunden lang auf 210 bis 220°C unter einem Druck von 6 bis 8 bar erhitzt. Das entstehende Ammoniak wurde über die aufgesetzte, bei 80 bis 85°C betriebene Kolonne unter nahezu totalem Rückfluss des n-Butanols aus der Reaktionslösung abgetrennt. Nach beendeter Reaktion wurde die Lösung in eine bei Normaldruck betriebene Füllkörperkolonne, an deren Kopfabzug 1923 g n-Butanol erhalten wurden, entspannt. Den Sumpf förderte man in eine bei ca. 160°C betriebene Strippkolonne, durch die ca. 300 Liter pro Liter Reaktionsgemisch und Stunde Stickstoff als Strippgas geblasen wurden. Über Kopf wurde ein Gemisch erhalten, das Carbamidsäurebutylester, Dibutylcarbonat und n-Butanol enthielt. Der Sumpf der Strippkolonne wurde ohne abzukühlen bei 30 mbar so in einen auf 270 bis 280°C erhitzten Dünnschichtverdampfer eingebracht, dass das Verhältnis an verdampftem 3-Butoxicarbonylaminomethyl-3,5,5-trimethyl-1-butoxicarbonylamino-cyclohexan zu ablaufender Schmelze ca. 4:1 war. Die Dämpfe führte man in einen Spaltreaktor mit ca. 3 Liter Leervolumen, der mit Messingringen von 3 mm Durchmesser gefüllt war. Die Temperatur im Spaltreaktor betrug durchschnittlich 410°C. Die austretenden Spaltgase wurden in einer nachgeschalteten zweistufigen Kondensationsvorrichtung fraktionierend kondensiert. Im ersten, bei 95°C betriebenen Kondensator erhielt man ein Gemisch aus 78 Gew.% Isophorondiisocyanat, 19 Gew.% einer Mischung aus 3-Butoxicarbonylaminomethyl-3,5,5-trimethyl-1-isocyanato-cyclohexan und 3-Isocyanatomethyl-3,5,5-trimethyl-1-butoxycarbonylamino-cyclohexan und 3 Gew.% 3-Butoxicarbonylaminomethyl-3,5,5-trimethyl-1-butoxicarbonylamino-cyclohexan; hieraus wurden in einer nachgeschalteten Destillation 1472 g Isophorondiisocyanat mit einer Reinheit >99% gewonnen.

Den Sumpf der Reindestillation vermischte man mit dem im zweiten, bei 10 bis 12°C betriebenen Kondensator erhaltenen Austrag, dem Ablauf des Dünnschichtverdampfers, dem Kopfprodukt der Strippkolonne und dem in der ersten Destillation gewonnenen n-Butanol und erhitzte diese Mischung zwei Stunden lang zum Sieden. Eine Analyse dieser Mischung mittels Gaschromatographie und Hochdruck-Flüssigchromatographie zeigte, dass in ihr 113 g Carbamidsäure-butylester, 102 g Dibutylcarbonat, 3151 g n-Butanol und 2066 g 3-Butoxicarbonylaminomethyl-3,5,5-trimethyl-1-butoxicarbonylamino-cyclohexan enthalten waren, was einer Selektivität von Isophorondiamin zu Isophorondiisocyanat von ca. 95% entsprach.

**Patentansprüche**

1. Mehrstufiges Verfahren zur Herstellung von 3-Isocyanatomethyl-3,5,5-trimethyl-cyclohexylisocyanat, dadurch gekennzeichnet, dass man

a) 3-Aminomethyl-3,5,5-trimethyl-cyclohexylamin mit Harnstoff und Alkohol in Gegenwart von Dialkylcarbonaten und/oder Carbamidsäurealkylestern sowie gegebenenfalls Katalysatoren zu 3-Alkoxicarbonylaminomethyl-3,5,5-trimethyl-1-alkoxicarbonylamino-cyclohexan umsetzt und das entstehende Ammoniak gleichzeitig abtrennt,

b) aus der erhaltenen Reaktionsmischung den Alkohol, die Dialkylcarbonate und/oder Carbamidsäurealkylester abtrennt und vorzugsweise in die Reaktionsstufe (a) zurückführt,

c) das 3-Alkoxicarbonylaminomethyl-3,5,5-trimethyl-1-alkoxicarbonylamino-cyclohexan in einem Verdampfer bei Temperaturen von 200 bis 300°C und einem Druck von 0,1 bis 200 mbar verdampft,

d) die Dämpfe bei Temperaturen von über 300°C und einem Druck von 0,1 bis 200 mbar in einem Spaltreaktor in 3-Isocyanatomethyl-3,5,5-trimethyl-cyclohexylisocyanat und Alkohol thermisch spaltet und

e) die Spaltprodukte fraktioniert kodensiert.

2. Mehrstufiges Verfahren zur Herstellung von 3-Isocyanatomethyl-3,5,5-trimethyl-cyclohexylisocyanat nach Anspruch 1, dadurch gekennzeichnet, dass die erhaltene Reaktionsmischung (b) aus 3-Alkoxicarbonylaminomethyl-3,5,5-trimethyl-1-alkoxicarbonylamino-cyclohexan, Dialkylcarbonat und/oder Carbamidsäurealkylester, Alkohol und gegebenenfalls Isophoron-oligoharnstoff-polyurethanen zweistufig getrennt wird, wobei

i) in der ersten Stufe der Alkohol bis auf einen Restalkoholgehalt von 1 bis 30 Gew.%, bezogen auf das Gesamtgewicht der Reaktionsmischung (b) abdestilliert und in die Reaktionsstufe (a) zurückgeführt wird und

ii) in der zweiten Stufe der restliche Alkohol, das Dialkylcarbonat und/oder der Carbamidsäurealkylester durch Strippen mit Inertgas vom 3-Alkoxicarbonylaminomethyl-3,5,5-trimethyl-1-alkoxicarbonylamino-cyclohexan und gegebenenfalls Isophoron-oligoharnstoff-polyurethan abgetrennt und in die Reaktionsstufe (a) zurückgeführt werden.

3. Mehrstufiges Verfahren zur Herstellung von 3-Isocyanatomethyl-3,5,5-trimethyl-cyclohexylisocyanat nach Ansprüchen 1 und 2, dadurch gekennzeichnet, dass der restliche Alkohol, das Dialkylcarbonat und/oder der Carbamidsäurealkylester in einer Strippkolonne bei Temperaturen von 50 bis 200°C mit 50 bis 5000 l Inertgas pro Liter Reaktionsgemisch und Stunde abgetrennt werden.

4. Mehrstufiges Verfahren zur Herstellung von 3-Isocyanatomethyl-3,5,5-trimethyl-cyclohexylisocyanat nach Anspruch 1, dadurch gekennzeichnet, dass man die Spaltprodukte in einer nachgeschalteten Zweistufenkondensationsvorrichtung fraktioniert kondensiert, wobei im ersten Teil der Kondensationsvorrichtung im wesentlichen 3-Isocyanatomethyl-3,5,5-trimethyl-cyclohexylisocyanat, das einer nachfolgenden Reindestillaton unterworfen wird und im zweiten Teil der Kondensationsvorrichtung im wesentlichen Alkohol kondensiert wird, der zusammen mit dem bei der Reindestillation des 3-Isocyanatomethyl-3,5,5-trimethyl-cyclohexylisocyanat erhaltenen Sumpfprodukt in die Reaktionsstufe (a) zurückgeführt wird.

5. Mehrstufiges Verfahren zur Herstellung von 3-Isocyanatomethyl-3,5,5-trimethyl-cyclohexylisocyanat nach Anspruch 1, dadurch gekennzeichnet, dass in der Reaktionsstufe (a) 3-Aminomethyl-3,5,5-trimethyl-cyclohexylamin mit Harnstoff und Alkohol im Molverhältnis 1:1,8 bis 2,5:2 bis 10 zur Reaktion gebracht wird.

6. Mehrstufiges Verfahren zur Herstellung von 3-Isocyanatomethyl-3,5,5-trimethyl-cyclohexylisocyanat nach Anspruch 1, dadurch gekennzeichnet, dass man in der Reaktionsstufe (a) als Alkohole n-Propanol, n-Butanol und/oder iso-Butanol verwendet.

7. Mehrstufiges Verfahren zur Herstellung von 3-Isocyanatomethyl-3,5,5-trimethyl-cyclohexylisocyanat nach Anspruch 1, dadurch gekennzeichnet, dass man in der Reaktionsstufe (a) den dem Alkohol entsprechenden Carbamidsäurealkylester in einer Menge von 1 bis 20 Mol.%, bezogen auf 3-Aminomethyl-3,5,5-trimethylcyclohexylamin verwendet.

8. Mehrstufiges Verfahren zur Herstellung von 3-Isocyanatomethyl-3,5,5-trimethyl-cyclohexylisocyanat nach Anspruch 1, dadurch gekennzeichnet, dass man in der Reaktionsstufe (a) das dem Alkohol entsprechende Dialkylcarbonat in einer Menge von 1 bis 30 Mol.%, bezogen auf 3-Aminomethyl-3,5,5-trimethyl-cyclohexylamin, verwendet.

9. Mehrstufiges Verfahren zur Herstellung von 3-Isocyanatomethyl-3,5,5-trimethyl-cyclohexylisocyanat nach Anspruch 1, dadurch gekennzeichnet, dass das in der Reaktionsstufe (a) gebildete Ammoniak mit Hilfe einer Destillationsvorrichtung bei Temperaturen von 60 bis 150°C von der Reaktionsmischung abgetrennt wird.

10. Mehrstufiges Verfahren zur Herstellung von 3-Isocyanatomethyl-3,5,5-trimethyl-cyclohexylisocyanat nach Anspruch 1, dadurch gekennzeichnet, dass man als Verdampfer einen Dünnschichtverdampfer verwendet und das 3-Alkoxicarbonylaminomethyl-3,5,5-trimethyl-1-alkoxicarbonylamino-cyclohexan und gegebenenfalls Isophoron-oligoharnstoff-polyurethan so zuführt, dass 20 bis 90 Gew.% 3-Alkoxicarbonylaminomethyl-3,5,5-trimethyl-1-alkoxicarbonylamino-cyclohexan verdampfen und 10 bis 80 Gew.%, zusammen mit gegebenenfalls vorliegendem Isophoron-oligoharnstoff-polyurethan ablaufen und in die Reaktionsstufe (a) zurückgeführt werden.

## Claims

1. A multiple-step process for the preparation of 3-isocyanatomethyl-3,5,5-trimethylcyclohexyl isocyanate, wherein

(a) 3-aminomethyl-3,5,5-trimethylcyclohexylamine is reacted with urea and alcohol in the presence of a dialkyl carbonate and/or carbamic acid alkyl ester and, if desired, a catalyst to form 3-alkoxycarbonylaminomethyl-3,5,5-trimethyl-1-alkoxycarbonylamino-cyclohexane, while the ammonia which forms is simultaneously removed,

(b) the alcohol, dialkyl carbonate and/or carbamic acid alky ester are separated from the resulting reaction mixture and, preferably, returned to reaction step (a),

(c) the 3-alkoxycarbonylaminomethyl-3,5,5-trimethyl-1-alkoxycarbonylaminocyclohexane is evaporated in an evaporator at a temperature of from 200 to 300°C and at a pressure of from 0.1 to 200 mbar,

(d) the vapors are thermally cleaved at a temperature in excess of 300°C and at a pressure of from 0.1 to 200 mbar in a cleaving reactor into 3-isocyanatomethyl-3,5,5-trimethylcyclohexyl isocyanate and alcohol, and

(e) the cleavage products are fractionally condensed.

2. A multiple-step process for the preparation of 3-isocyanatomethyl-3,5,5-trimethylcyclohexyl isocyanate as claimed in claim 1, wherein the resulting reaction mixture (b) of 3-alkoxycarbonylaminomethyl-3,5,5-trimethyl-1-alkoxycarbonylaminocyclohexane, dialkyl carbonate and/or carbamic acid alkyl ester, alcohol and, possibly, isophorone/oligourea polyurethanes is separated in two steps, whereby

(i) in the first step, the alcohol is distilled off until the residual alcohol content is from 1 to 30 percent by weight, based on the total weight of the reaction mixture (b), and the distilled alcohol is returned to reaction step (a), and

(ii) in the second step, the residual alcohol, dialkyl carbonate and/or carbamic acid alkyl ester are separated from the 3-alkoxycarbonylaminomethyl-3,5,5-trimethyl-1-alkoxycarbonylaminocyclohexane and any isophorone/oligourea polyurethanes by stripping with inert gas, and returned to reaction step (a).

3. A multiple-step process for the preparation of 3-isocyanatomethyl-3,5,5-trimethylcyclohexyl isocyanate as claimed in claims 1 and 2, wherein the residual alcohol, dialkyl carbonate and/or carbamic acid alkyl ester are separated off in a stripping column at a temperature of from 50 to 200°C with 50 to 5000 l of inert gas per liter of reaction mixture per hour.

4. A multiple-step process for the preparation of 3-isocyanatomethyl-3,5,5-trimethylcyclohexyl isocyanate as claimed in claim 1, wherein the cleavage products are fractionally condensed in a downstream two-stage condenser, 3-isocyanatomethyl-3,5,5-trimethylcyclohexyl isocyanate being primarily condensed in the first stage, which isocyanate is subsequently subjected to a refining distillation, and alcohol being primarily condensed in the second stage of the condenser, which alcohol is returned to reaction step (a) together with the bottoms product obtained in the refining distillation 3-isocyanatomethyl-3,5,5-trimethylcyclohexyl isocyanate.

5. A multiple-step process for the preparation of 3-isocyanatomethyl-3,5,5-trimethylcyclohexyl isocyanate as claimed in claim 1, wherein, in reaction step (a), 1.8 to 2.5 moles of urea and 2 to 10 moles of alcohol are reacted per mole of 3-aminomethyl-3,5,5-trimethylcyclohexylamine.

6. A multiple-step process for the preparation of 3-isocyanatomethyl-3,5,5-trimethylcyclohexyl isocyanate as claimed in claim 1, wherein the alcohol used in reaction step (a) is n-propanol, n-butanol and/or isobutanol.

7. A multiple-step process for the preparation of 3-isocyanatomethyl-3,5,5-trimethylcyclohexyl isocyanate as claimed in claim 1, wherein, in reaction step (a), the carbamic acid alkyl ester corresponding to the alcohol is used in an amount of from 1 to 20 mole percent, based on 3-aminomethyl-3,5,5-trimethylcyclohexylamine.

8. A multiple-step process for the preparation of 3-isocyanatomethyl-3,5,5-trimethylcyclohexyl isocyanate as claimed in claim 1, wherein, in reaction step (a), the dialkyl carbonate corresponding to the alcohol is used in an amount of from 1 to 30 mole percent, based on 3-aminomethyl-3,5,5-trimethylcyclohexylamine.

9. A multiple-step process for the preparation of 3-isocyanatomethyl-3,5,5-trimethylcyclohexyl isocyanate as claimed in claim 1, wherein the ammonia formed in reaction step (a) is separated from the reaction mixture with the aid of a distillation device at a temperature of from 60 to 150°C.

10. A multiple-step process for the preparation of 3-isocyanatomethyl-3,5,5-trimethylcyclohexyl isocyanate as claimed in claim 1, wherein an agitated thin-film evaporator is used as the evaporator, and the 3-alkoxycarbonylaminomethyl-3,5,5-trimethyl-1-alkoxycarbonylaminocyclohexane and any isophorone/oligourea polyurethanes are added in such a manner that from 20 to 90 percent by weight of the 3-alkoxycarbonylaminomethyl-3,5,5-trimethyl-1-alkoxycarbonylaminocyclohexane evaporates and 10 to 80 percent by weight, together with any isophorone/oligourea polyurethanes which may be present, runs off and is returned to reaction step (a).

**Revendications**

1. Procédé à étapes multiples de préparation de l'isocyanate de 3-isocyanatométhyl-3,5,5-triméthyl-cyclohexyle, caractérisé en ce que

a) On convertit la 3-aminométhyl-3,5,5-triméthyl-cyclohexylamine avec l'urée et un alcool, en présence de carbonates de dialkyle et/ou d'esters alkyliques de l'acide carbamique, comme éventuellement aussi de catalyseurs, en 3-alcoxy-carbonylaminométhyl-3,5,5-triméthyl-1-alcoxycarbonylamino-cyclohexane et on sépare simultanément l'ammoniac engendré,

b) On sépare l'alcool, les carbonates de dialkyle et/ou les esters alkyliques de l'acide carbamique du mélange réactionnel obtenu et on les renvoie, de préférence à l'étape réactionnelle (a),

c) On évapore le 3-alcoxycarbonylaminométhyl-3,5,5-triméthyl-1-alcoxycarbonylaminocyclohexane à des températures de 200 à 300°C et sous une pression de 0,1 à 200 mbars dans un évaporateur,

d) On scinde thermiquement les vapeurs à des températures supérieures à 300°C et sous une pression de 0,1 à 200 mbars, en isocyanate de 3-isocyanatométhyl-3,5,5-triméthylcyclohexyle et en alcool dans un réacteur de scission, et

e) On condense les produits de scission sous fractionnement.

2. Procédé à étapes multiples de préparation de l'isocyanate de 3-isocyanatométhyl-3,5,5-triméthyl-cyclohexyle suivant la revendication 1, caractérisé en ce que l'on sépare le mélange réactionnel (b) obtenu constitué de 3-alcoxycarbonylami-

nométhyl-3,5,5-triméthyl-1-alcoxycarbonylamino-cyclohexane, de carbonate de dialkyle et/ou d'ester alkylique de l'acide carbamique, d'alcool et éventuellement d'isophorono-oligouréo-polyuréthanes en deux stades, suivant lesquels

i) au cours du premier stade, on sépare l'alcool jusqu'à une teneur en alcool résiduel de 1 à 30% en poids par rapport au poids global du mélange réactionnel (b), par distillation et on le renvoie dans l'étape réactionnelle (a), et

ii) au cours du second stade, on sépare l'alcool résiduel, le carbonate de dialkyle et/ou l'ester alkylique d'acide carbamique par lavage (stripage) avec un gaz inerte, du 3-alcoxycarbonylaminométhyl-3,5,5-triméthyl-1-alcoxycarbonylamino-cyclohexane et éventuellement de l'isophorono-oligouréo-polyuréthane et on les renvoie à l'étape réactionnelle (a).

3. Procédé à étapes multiples de préparation de l'isocyanate de 3-isocyanatométhyl-3,5,5-triméthyl-cyclohexyle suivant la revendications 1 et 2, caractérisé en ce que l'on sépare l'alcool résiduel, le carbonate de dialkyle et/ou l'ester alkylique de l'acide carbamique dans une colonne de stripage, à des températures de 50 à 200°C, avec 50 à 5000 l de gaz inerte par litre de mélange réactionnel et par heure.

4. Procédé à étapes multiples de préparation de l'isocyanate de 3-isocyanatométhyl-3,5,5-triméthyl-cyclohexyle suivant la revendication 1, caractérisé en ce que l'on condense sous fractionnement les produits de la scission (séparation) dans un dispositif de condensation à deux stades (étages) successifs, où dans la première partie du dispositif de condensation, on condense essentiellement l'isocyanate de 3-isocyanatométhyl-3,5,5-triméthyl-cyclohexyle que l'on soumet à une distillation de purification subséquente et, dans la seconde partie du dispositif de condensation, on condense essentiellement l'alcool que l'on renvoie, en même temps que le produit de fond obtenu au cours de la distillation de purification de l'isocyanate de 3-isocyanatométhyl-3,5,5-triméthyl-cyclohexyle, à l'étape réactionnelle (a).

5. Procédé à étapes multiples de préparation de l'isocyanate de 3-isocyanatométhyl-3,5,5-triméthyl-cyclohexyle suivant la revendication 1, caractérisé en ce que, au cours de l'étape réactionnelle (a) on fait réagir la 3-aminométhyl-3,5,5-triméthylcyclohexylamine sur l'urée et l'alcool dans un rapport molaire 1:1,8 à 2,5:2 à 10.

6. Procédé à étapes multiples de préparation de l'isocyanate de 3-isocyanatométhyl-3,5,5-triméthyl-cyclohexyle suivant la revendication 1, caractérisé en ce que l'on utilise le n-propanol, le n-butanol et/ou l'isobutanol à titre d'alcools au cours de l'étape réactionnelle (a).

7. Procédé à étapes multiples de préparation de l'isocyanate de 3-isocyanatométhyl-3,5,5-triméthyl-cyclohexyle suivant la revendication 1, caractérisé en ce que l'on utilise l'ester alkylique d'acide carbamique correspondant à l'alcool en une proportion de 1 à 20% molaires par rapport à la 3-aminométhyl-3,5,5-triméthyl-cyclohexylamine, au cours de l'étape réactionnelle (a).

8. Procédé à étapes multiples de préparation de l'isocyanate de 3-isocyanatométhyl-3,5,5-triméthyl-cyclohexyle suivant la revendication 1, caractérisé en ce que l'on utilise le carbonate de dialkyle correspondant à l'alcool en une proportion de 1 à 30 molaires par rapport à la 3-aminométhyl-3,5,5-triméthyl-cyclohexylamine, au cours de l'étape réactionnelle (a).

9. Procédé à étapes multiples de préparation de l'isocyanate de 3-isocyanatométhyl-3,5,5-triméthyl-cyclohexyle suivant la revendication 1, caractérisé en ce que l'on sépare l'ammoniac formé au cours de l'étape réactionnelle (a) du mélange réactionnel, à des températures de 60 à 150°C, à l'aide d'un dispositif de distillation.

10. Procédé à étapes multiples de préparation de l'isocyanate de 3-isocyanatométhyl-3,5,5-triméthyl-cyclohexyle suivant la revendication 1, caractérisé en ce que l'on utilise un évaporateur à couche mince à titre d'évaporateur et en ce que l'on introduit le 3-alcoxycarbonylaminométhyl-3,5,5-triméthyl-1-alcoxycarbonylamino-cyclohexane et éventuellement l'isophorono-oligouréo-polyuréthane de façon à ce que de 20 à 90% en poids du 3-alcoxycarbonylaminométhyl-3,5,5-triméthyl-1-alcoxycarbonylamino-cyclohexane s'évaporent et que de 10 à 80% en poids de ce composé s'écoulent avec l'isophorono-oligouréo-polyuréthane éventuellement présent, pour être renvoyés à l'étape réactionnelle (a).